# EUROPEAN PATENT APPLICATION

(11) **EP 0 561 439 A2**
(43) Date of publication of application: **22.09.1993**
(21) Application number: 93200430.2
(22) Date of filing: 15.02.1993
(51) Int. Cl.: B01J 8/12, C07C 5/333, B01J 19/22

(54) **Process and apparatus for performing chemical conversions**

(30) Priority: 17.02.1992 EP 92200446
(71) Applicant: SHELL INTERNATIONALE RESEARCH MAATSCHAPPIJ B.V., NL-2596 HR Den Haag (NL)
(72) Inventor: van Diepen, Petrus Bernardus Josef, NL-1031 CM Amsterdam (NL); Veenstra, Peter, NL-1031 CM Amsterdam (NL); Groeneveld, Michiel Jan, durong, 97008 Bintulu, Sarawak (MA); Arnoldy, Peter, NL-1031 CM Amsterdam (NL); Wolff, Ericus Henricus Petrus, NL-1031 CM Amsterdam (NL); Dierickx, Jan Lodewijk Maria, NL-1031 CM Amsterdam (NL)

(57) **Abstract**

A process and apparatus for performing chemical conversions, in which one or more gaseous organic reactants are contacted at an elevated temperature with solid particles in a cross-flow moving bed reactor, the bed of solid particles moving in a direction inclined from 1 to 30 degrees downward from the horizontal, the reactant gases being introduced into the moving bed from below through a gas-permeable wall of the reactor and the effluent reaction products leaving the moving bed from above.

## Description

The invention relates to a process for performing chemical conversions whereby gaseous organic reactants are contacted with solid materials at elevated temperatures.

The invention also relates to an apparatus suitable to be used in the above process.

In the art many chemical processes are described which, for various reasons, involve an intimate contact between the feed to be converted and solid materials. These processes include thermal processes whereby the solid material mainly serves to ensure adequate contact between reacting molecules and to transfer heat to, or from, the reactants or products. They also include catalytic processes whereby the solid material has catalytic activity for the reaction at issue.

Examples of processes whereby the use of solid contacting material has been proposed, are thermal and catalytic cracking processes, oxidation processes, such as the oxidation of n-butane to maleic anhydride and the oxidation of propene and ammonia to acrylonitrile, and various dehydrogenation processes.

For some of these processes, the conversions achieved at moderate temperatures are often too low to make the process economically viable, even if use is made of a catalyst.

At higher temperatures an adequate conversion of the starting material can usually be achieved, but then often problems are encountered as regards the selectivity with respect to the desired product(s).

This may be caused by the occurrence of side reactions which, at the said high temperatures, are no longer negligible and result in the formation of significant amounts of undesired by-products. Also sequential reactions may take place, whereby the initially formed desired product acts as an intermediate which is readily converted into unwanted products such as coke.

In view of the importance of some of the processes concerned, the realisation of improved results would be desirable.

In particular for a number of dehydrogenation processes, their commercial potential has stimulated the efforts to come to an operation of the process whereby the desired product is obtained in high yield in an economically viable manner.

Various operational procedures have been tried aiming to attain both an adequate conversion of the feed and a sufficient selectivity towards the desired product. However, although some improvements have indeed been made, it appears that the advantages obtainable by using a particular mode of operation, are counteracted by serious disadvantages in another aspect.

Thus in US-A. 4,607,129 a dehydrogenation process is described whereby the hydrocarbon feed is contacted with solid catalyst in fixed bed operation. With that type of operation an adequate temperature balance is difficult to achieve. At the temperatures applied the obtained conversion is relatively low. At higher temperatures, this procedure results in considerable coke formation. Similar problems are encountered when the process is performed in a (slowly, e.g. of over one hour) moving bed operation.

According to another procedure, proposed for dehydrogenation processes, the feed is introduced into a fluidized bed of catalyst particles. In this procedure an adequate catalyst hold-up and a good heat transfer is achieved, but a disadvantage is that gas backmixing may occur. In order to maintain a short gas residence time, needed to prevent occurrence of undesired side reactions, the height of the catalyst bed will have to remain low, with the consequential risk of channelling of gas through the bed, so that the obtainable conversion remains low.

Also the use of riser reactors does not lead to satisfactory results, because catalyst hold-up in this type of reactors is usually too low. And at higher catalyst hold-up, the high catalyst/feed ratios prevailing require the supply of excessive amounts of stripping agent, usually steam, to strip the catalyst, before the latter is reused and attrition problems are often encountered.

It has now been found that chemical conversions of the present category, i.e. in particular chemical processes requiring short gas residence times, (much) longer solids residence times and high solids hold-up without having to apply excessive amounts of stripping gas, are successfully carried out by making use of a particular type of cross-flow moving bed (CFMB) reactor allowing the above conditions to be simultaneously fulfilled.

Cross-flow moving beds as such are known as useful equipment for substantially horizontal transport of powdery materials.

In EP. 122925 a closed device is described for conveying powdery materials from a storage area to one or more supply areas. The device comprises a horizontal or inclined conveyor provided with fluidization means, having a lower gas-circulation channel and an upper channel separated by a porous wall from the lower channel. Gas is introduced from the lower channel to the upper channel via the said porous wall. The device is characterized in that, in order to potentially fluidize the powder in the conveyor, the latter is provided with one or more balancing columns of which the filling height balances the pressure of the potential fluidization gas.

JP-51-129871 (KOKAI) discloses a method for the removal of dust and oxides of nitrogen from combustion waste gases, in which the gases are introduced from above in a reactor wherein a catalyst bed is moved horizontally on a porous conveyor by mechanical means, such as rollers.

Surprisingly, a device as described in EP-122925 can be successfully used as a reactor in performing chemical conversions with gaseous organic reactants.

The present invention therefore concerns a process for performing chemical conversions, in which one or more gaseous organic reactants are contacted at an elevated temperature with solid particles in a cross-flow moving bed reactor, the bed of solid particles moving in a direction inclined from 1 to 30 degrees downward from the horizontal, the reactant gases being introduced into the moving bed from below through a gas-permeable wall of the reactor and the effluent reaction products leaving the moving bed from above.

Preferably, the downward inclination is from 3 to 10 degrees.

The CFMB reactor can be used for processes whereby the solid particles in the moving bed merely promote the contact between the gaseous reactants and hence facilitate the reaction to proceed. However, preferably use is made of solid materials exhibiting catalytic activity for the reaction envisaged.

An important advantage of the use of a CFMB reactor is that the gas residence time can be preselected and easily readjusted if so desired, independent of the solids residence time.

The invention is of particular interest to be used for the dehydrogenation of organic compounds which are gaseous under the reaction conditions and which have in their molecules at least one hydrogen atom linked to each of two adjacent carbon atoms.

Suitable examples of such compounds are hydrocarbons having from 2 to 6 carbon atoms, e.g. ethane, propane, butane, isobutane and hexane. Propane and isobutane are in particular preferred starting materials. The dehydrogenated products thus obtained are widely used in the industry. Propene represents a valuable monomer in polymerization processes, e.g. in the manufacture of polypropylene. Isobutene is a feedstock for the production of methyl tertiary butyl ether which is applied as octane boosting component for gasoline.

Furthermore the process of the invention can be suitably applied for oxidation reactions such as the oxidation of propene and ammonia to acrylonitrile, and the oxidation of n-butane to maleic anhydride.

Solid materials exhibiting catalytic activity for the above mentioned dehydrogenation processes may substantially consist of the catalytically active compounds, but usually consist of inert carrier material on which the catalytically active compounds or precursors thereof are supported.

Suitable carrier materials include refractory supports such as those comprising aluminates or oxides of magnesium, aluminium, silicon or titanium. Preferred carrier materials comprise silica and/or alumina, in particular gamma alumina.

Suitable catalytically active compounds include platinum and oxides of vanadium, iron, chromium and zinc. Platinum and oxides of vanadium and chromium are preferred.

According to an advantageous method for preparing the solid, catalytically active particles, carrier material is impregnated with a solution of the selected catalytically active compound or a precursor thereof in a suitable solvent, followed by removal of the solvent, e.g. by drying. Subsequently, a heat treatment may be carried out e.g. at a temperature in the range from 300 to 800 °C, preferably in a controlled oxidative or reductive atmosphere. A similar heat treatment may be applied in regenerating or reactivating catalytic material used in the process.

Shape and size of the solid particles can vary considerably, albeit that in order to be suitable for use in a CFMB reactor, there are some limitations. Substantially spherical-shaped particles are preferred. Suitable average particle diameters usually are in the range of 0,05x10-³ m to 10x10-³ m, preferably in the range of 0,3x10-³ m to 3x10-³ m.

When in operation for performing one of the above mentioned chemical conversions, the flow of gas through the CFMB reactor can conveniently arranged to be of the plug flow (or laminar flow) type. This is of advantage because during operation there will be virtually no gas back-mixing which could have a negative effect on the selectivity and yield.

In particular for the aforesaid catalytic processes, the gas residence time in the CFMB reactor is kept short, as otherwise the occurrence of side and/or sequential reactions such as coke formation could become a problem. Usually a gas residence time is chosen of less than 1 min. and often considerably less, e.g. in the range from 0.005 to 5 sec. A gas residence time of from 0.1 to 3 seconds is preferred.

The residence time of the solid particles in the CFMB reactor is also kept relatively short, although it may be somewhat longer than the gas residence time. Usually a solid particle residence time is chosen of less than 10 minutes, and often considerably less, e.g. in the range of from 5 to 100 seconds.

Another advantage of using a CFMB reactor resides in the possibility of achieving a high solids hold-up. In the event that the solid material has catalytic activity, a high solids hold-up enables a maximal conversion. Depending on such factors as the height of the bed in the reactor, the slope and the length of the reactor, a certain (high) solids hold-up will result which, if desired, may be optimized by adjusting one or more of the said factors.

A further advantage of the CFMB reactor resides in the possibility it opens for controlling the reaction temperature by varying the temperature of the gas stream introduced along the reactor. Thus, when the reaction is exothermic the working temperature inside the reactor can be kept constant by introducing the reactant gas at a declining temperature.

The flow at which the gaseous reactants are introduced is preferably selected up to or around the minimal fluidization velocity, that is the gas velocity whereby under the reaction conditions a fluidized bed may just be formed. Higher flows than those necessary to initiate fluidization, are preferably not used, because at higher pressure bubble forming tends to occur. Formation of bubbles will be at the expense of intimate contact between gas and solid material and hence often at the expense of obtainable conversion and yield.

Suitable pressures usually are in the range of 0.1 to 100 bar, preferably in the range of 0.2 to 5 bar, in particular of 1.5 to 4 bar.

The solid particles and the gaseous reactants may conveniently be at or near the reaction temperature when they are introduced in the reactor. Additional heat may be supplied to, or in the event of exothermal conversions, be removed from the reaction mixture.

For most chemical conversions to which the invention relates, and in particular for dehydrogenation reactions, the reaction temperature is between 200 and 850 °C. In some cases lower or higher temperatures may be applied, but preferably the CFMB reactor is operated at a temperature between 550 and 750 _{°} C.

Figure I is a flow scheme, illustrating a preferred embodiment of the invention:

A rectangular reactor (1) of the CFMB type is operated by introducing solid particles at the top- end of the reactor from a bunker (2) and gaseous reactants via a porous wall (not shown) extending over the length of the reactor. The solid particles leaving the reactor are fed into a zone (3) from which the entrained gaseous reaction products are recovered. Preferably (3) is a stripping zone. For the removal of dehydrogenated hydrocarbons from the reaction mixture, steam is a preferred stripping agent.

The solid particles leaving zone (3) which are usually contaminated with deposits such as carbonaceous material are preferably regenerated and subsequently recycled to the reactor. If the solid particles exhibit catalytic activity, this activity may have diminished during the reaction, so that regeneration in an oxidative atmosphere is desirable.

For the regeneration of the solid particles use may be made of a second CFMB reactor (4), since intimate contact between the loaded particles and the regenerative gas, usually an oxygen containing gas, such as air, is then guaranteed.

The regenerated particles are preferably subjected to a stripping treatment in a second stripping zone (5), in order to remove remaining oxygen. Steam is a preferred stripping agent. The regenerated stripped particles are conveniently transported via a pneumatic transport system (6) to a separator (7), from which they are recycled into the bunker (2).

The invention also concerns an apparatus suitable for performing chemical conversions, comprising a cross-flow moving bed reactor inclined in the flow direction between 1 to 30 degrees downwards from the horizontal and having a gas-permeable lower wall, and means for regenerating and recycling solid particles removed from the reactor during operation.

The process of the invention is illustrated with the following Examples.

### Example 1, dehydrogenation of isobutene.

### a) Preparation of the catalyst.

Carrier particles, consisting of 1.5 mm particles of gamma-alumina with a surface area of 259 m²/g were impregnated in two stages.

In the first stage the carrier particles, 85 g, were impregnated with a solution of 3.35 g SnS0₄/1 ml 96 % H₂S0₄ in 30 ml water, made up to 60 ml with additional water and then shaked for 1 hour.

During 4 hours the mixture was dried at 120 °C followed by a calcination treatment at 500 °C during 57 hours.

In the second stage the calcined particles were impregnated with two portions of a solution of Cr0₃ and CₛNO₃ in water, obtained by mixing 21.23 g Cr0₃/25 ml H₂0 with 2.98 g C_{S}N0₃/20 ml H₂0 made up to 134 ml with additional water, and subsequent mild warming up. After addition of the first portion a drying treatment of 1 hour at 120 °C occurred and, after addition of the second portion, another drying treatment of 4 hours, again at 120 °C. Subsequently the particles were calcined for 16 hours at 500 °C.

The catalyst thus prepared, contained 1.8 %w Sn, 10.3 %w Cr and 1.9 %w Cs, calculated on total weight.

### b) The dehydrogenation reaction.

A rectangular CFMB reactor (as in Figure I, 1) with a bed volume of 8.7 and a length of 7.2, which is inclined 6 degrees downward, is charged from a bunker (2) at a rate of 436 parts by weight/s with catalyst particles, which had been prepared as stated above. Isobutane is introduced through the porous bottom wall of the reactor, at a rate of 21.8 parts by weight/s and a pressure of 2.2 bar. The temperature in the reactor is 630 °C, and the pressure 2.1 bar. The residence time of the gas reactant in the reactor is 0.3 seconds, and the residence time of the catalyst particles is 14 seconds.

The catalyst particles leaving the reactor are charged to a stripper (3) with a volume of 8.3 wherein hydrocarbons are stripped off with 4.4 parts by weight/s of steam at a temperature of 630 _{°} C.

Products from the reactor and stripper are quenched with pye gas at 220 _{°} C.

Analysis of the product mixture by gas-liquid chromatography shows that 11.05 parts by weight/s isobutene had been formed (51 % yield) together with some n-butene (0.55 parts by weight/s), propane (0.5 parts by weight/s), methane (0.37 parts by weight/s), and propene (0.35 parts by weight/s). In addition, 0.83 parts by weight of coke is formed. Unconverted isobutane amounts to 7.39 parts by weight/s.

The solid particles leaving the stripper are regenerated in a second CFMB rectangular reactor (4) with a volume of 11.0 and a length of 9.3, wherein with the aid of an air stream (14.9 parts by weight/s at a pressure of 2.4 bar) at 660 _{°} C coke is burnt off under formation of C0₂ (3.04 parts by weight/s).

The regenerated solid particles are transferred into a second stripper (5) with a volume of 8.3 wherein with the aid of steam (4.1 parts by weight/s) at a temperature of 690 _{°} C and a pressure of 2.4 bar) oxygen is stripped off.

The catalyst particles are subsequently transported to the bunker (2) for reuse in the process.

### Example 2, oxidation of n-butane to maleic acid.

A rectangular CMFB reactor (as in Figure I, 1) with a bed volume of 7.5, a length of 3.6 and a width of 2.0, which is inclined 6 degrees downward, is charged from a bunker (2) with oxidized vanadium-phosphorus-oxide spherical catalyst particles (average diameter 1.6 mm, apparent density 1600 kg/_{M}³), at a rate of 853 parts by weight/s. b-Butane is introduced through the porous bottom wall of the reactor, at a rate of 1.7 parts by weight and a pressure of 2.2 bar. The residence time of the gas reactant in the reactor is 2.2 seconds, and the residence time of the catalyst particles is 7 seconds.

The particles leaving the reactor are charged to a stripper (3) with a volume of 10.5, wherein hydrocarbons are stripped off with 6.0 parts by weight/s of steam at a temperature of 360 _{°} C and a pressure of 2.4 bar.

The solid particles leaving the stripper are re-oxidised in a second rectangular CMFB reactor (4) with a volume of 30.8, a length of 14.4 and a width of 2.1, wherein molecular oxygen is stripped off with the aid of an air stream (28.5 parts by weight/s at a pressure of 2.1 bar at 360 _{°} C).

The re-oxidised solid particles are transferred to a second stripper (5) with a volume of 10.5, wherein molecular oxygen is stripped off with the aid of steam (6.0 parts by weight/s, temperature 360 _{°} C, pressure 2.4 bar).

The catalyst particles are subsequently transported to the bunker (2) for reuse in the process.

The relationship between the volumina, lengths and parts by weight given in the above Examples (1 b and 2) is as between m³, m and kg.

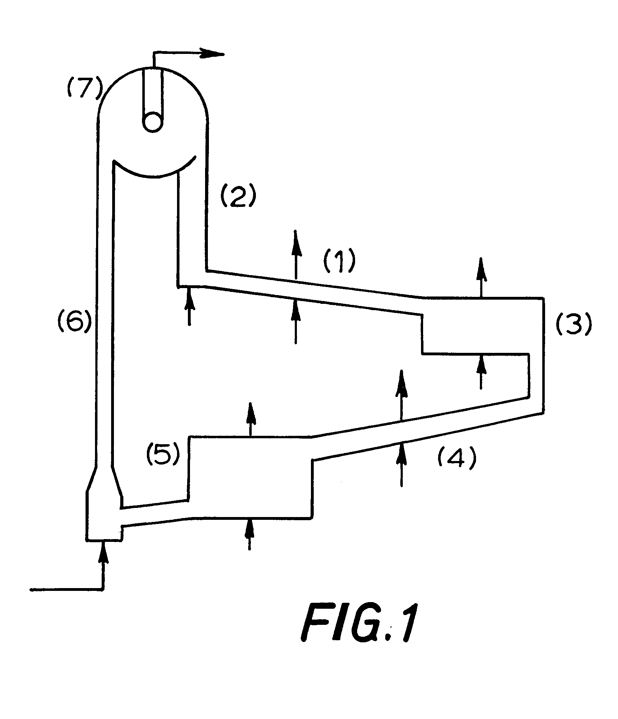

## Claims

1. A process for performing chemical conversions, in which one or more gaseous organic reactants are contacted at an elevated temperature with solid particles in a cross-flow moving bed reactor, the bed of solid particles moving in a direction inclined from 1 to 30 degrees downward from the horizontal, the reactant gases being introduced into the moving bed from below through a gas-permeable wall of the reactor and the effluent reaction products leaving the moving bed from above.

2. A process according to claim 1, characterized in that the solid particles comprise material which is catalytically active in the reaction.

3. A process according to claim 1 or 2, characterized in that the gases are introduced at a flow rate of around the minimal fluidization velocity.

4. A process according to any one of claims 1-3, characterized in that the residence time of the gases in the moving bed reactor is from 0.1 to 3 seconds.

5. A process according to any one of claims 1-4, characterized in that the residence time of the solid particles in the moving bed reactor is from 5 to 100 seconds.

6. A process according to any of claims 1-5, characterized in that the pressure in the reactor is in the range of 0.2 to 5 bar.

7. A process according to any one of claims 1-6, characterized in that the contact temperature in the reactor is in the range of 200 to 850 _{°} C.

8. A process according to any one of claims 1-7, characterized in that the solid particles leaving the reactor are stripped in a first stripping zone to remove effluent reaction products, regenerated in a regeneration zone and recycled to the reactor.

9. A process according to claim 8, characterized in that the solid particles are regenerated in the regeneration zone with an oxygen containing gas and subsequently stripped in a second stripping zone.

10. A process according to any one of claims 1-9, characterized in that as the gaseous organic reactants one or more compounds having at least one hydrogen atom linked to each of two adjacent carbon atoms are dehydrogenated.

11. A process according to claim 10, characterized in that the gaseous organic compound is propane or isobutane.

12. A process according to claim 10 or 11, characterized in that the solid particles comprise platinum or vanadia supported on a refractory support.

13. A process according to any one of claims 1 - 9, characterized in that n-butane is oxidised.

14. Apparatus suitable for performing chemical conversions, comprising a cross-flow moving bed reactor inclined in the flow direction between 1 to 30 degrees downwards from the horizontal and having a gas-permeable lower wall, and means for regenerating and recycling solid particles removed from the reactor during operation.
